# EUROPEAN PATENT SPECIFICATION

(11) **EP 1 968 777 B1**
(45) Date of publication and mention of the grant of the patent: **06.11.2013**
(21) Application number: 06847844.5
(22) Date of filing: 20.12.2006
(51) Int. Cl.: B29C 45/14, B29C 45/02, A61M 37/00

(54) **MANUFACTURING MICRONEEDLE ARRAYS**
HERSTELLUNG VON MIKRONADELANORDNUNGEN
FABRICATION DE MATRICES DE MICROAIGUILLES

(30) Priority: 23.12.2005 US 753808 P
(43) Date of publication of application: 17.09.2008
(73) Proprietor: 3M Innovative Properties Company, St. Paul, MN 55133-3427 (US)
(72) Inventor: FERGUSON, Dennis E., Saint Paul, MN 55133-3427 (US)
(74) Representative: Vossius & Partner
(86) International application number: PCT/US2006/048640
(87) International publication number: WO 2007/075806

(56) References cited:
- WO-A1-2005/082596
- US-A1- 2004 007 796
- US-A1- 2004 007 796
- US-B1- 6 334 856
- US-B1- 6 589 202
- US-B1- 6 589 202

## Description

### Field

The present invention relates to microstructured drug delivery devices, in particular injection molded microneedle articles and methods of making the same.

### Background

Molded plastic articles are well known and commonly used in everyday life. Most molded articles are relatively large in nature and/or are relatively rugged, and thus may be handled quite conveniently. Certain molded articles, however, are very small, include very fine microstructured features, and/or are otherwise sensitive in some respect, and thus may be difficult to handle conveniently. One example of such articles are arrays of relatively small structures, sometimes referred to as microneedles or micro-pins, which have been disclosed for use in connection with the delivery of therapeutic agents and other substances through or into the skin. The devices are typically pressed against the skin in an effort to pierce the stratum corneum such that the therapeutic agents and other substances can pass through that layer and into the tissues below.

A microneedle drug delivery device can be manufactured using a variety of methods. One method that has been proposed is by injection molding. An injection molded microneedle drug delivery device differs from many articles in that it is highly sensitive in many respects and will normally require further processing or treatment steps (e.g., application of coatings, surface treatments, combination into a larger device, packaging, etc.) once formed and may thus require further handling.

Document US-B-6589202 discloses a method of making a microstructured drug delivering article on a carrier web comprising the step of providing a web having a first carrier surface and a second back carrier surface.

### Summary of the Invention

Injection molded articles having microstructures for drug delivery, such as microneedles, are typically quite delicate and may be easily damaged or contaminated during normal handling. In particular, a number of intermediate handling steps are often necessary to take a molded microstructured article, such as a microneedle array, fashion it into a finished product, and deliver such a product to an end-use customer. The present invention relates to articles and methods useful for handling and/or protecting microstructured injection molded articles, including those having highly sensitive surfaces, such as microstructures, coatings, and surfaces that must remain undamaged and uncontaminated by contacts. It is also highly useful where the injection molded article is to have any further processing requiring the article to be in a fixed orientation, such as for coating, spraying, cleaning, machining, stamping, marking, inspecting, covering, packaging, curing, and the like.

The invention provides a method of making a molded microstructured drug delivery or tissue fluid sampling articles on a carrier web. A web having a first, carrier surface and a second, back surface is provided. A mold apparatus comprising an injection gate and a mold insert having microstructured features, wherein the mold apparatus has an open position and a closed position is provided. The mold apparatus is placed in the open position, a portion of the web is placed within an opening in the mold apparatus, and the mold is closed on the web.

Polymeric material is injected through the injection gate(s) into the closed mold apparatus to form a molded part that is affixed to the first, carrier surface of the web, wherein the molded part has microstructured features extending away from the carrier surface. The mold is opened and the molded part is removed from the mold insert. The web is advanced such that the molded part is moved outside of the mold apparatus and another portion of the web is placed within the opening in the mold apparatus.

It should also be noted in connection with the above aspects of the invention that the injection molded article can have a variety of highly sensitive microstructured features, such as at least one drug delivery needle or a plurality of drug delivery microneedles. The article may, after molding, be subsequently provided with a drug substance while the molded article remains affixed to the first, carrier surface of the web. The molded article may be further conveyed while affixed to the first, carrier surface of the web to a packaging process. The molded article may have no runner or sprue. The molded article may be pulled from the mold by applying tensile force to the web. The molded article may be conveyed while affixed to the first, carrier surface of the web to a curing or drying process. The molded article may be conveyed while remaining affixed to the first, carrier surface of the web by air or fluid conveyance of the web. The molded article may be conveyed while affixed to the first, carrier surface of the web to an inspection process. The web may be rolled into roll form with a plurality of molded articles affixed to the first, carrier surface of the web, and a protective cover liner may be applied to the molded articles prior to rolling the web into roll form.

As used herein, certain terms will be understood to have the meaning set forth below:
"Web" refers to a sheet material of indefinite length. In general, a sheet of web material has width and length dimensions much greater than the thickness of the sheet. The longitudinal or machine direction of the web refers to the general direction of motion of the web material through the molding and web handling apparatus. The transverse or cross direction of the web refers to a direction perpendicular to the longitudinal web direction.

"Microstructure" or "microstructured" refers to specific microscopic features or structures associated with a larger article. By way of example, microstructures can include projections and/or cavities on a surface of a larger article. Such microscopic features will generally have at least one dimension (e.g., length, width, height) that is about 500 microns or less in size.

"Array" or "microarray" refers to medical devices such as the type described herein that include one or more structures, such as a plurality of microneedles, extending from a substrate and capable of piercing the stratum corneum to facilitate the transdermal (including intradermal) delivery of therapeutic agents or the sampling of fluids through or into the skin.

"Microneedle" refers to a specific microscopic structure associated with the array that is designed for piercing the stratum corneum to facilitate the transdermal delivery of therapeutic agents or the sampling of fluids through the skin. By way of example, microneedles can include needle or needle-like structures, including microblades, as well as other structures capable of piercing the stratum corneum.

The features and advantages of the present invention will be understood upon consideration of the detailed description of the preferred embodiment as well as the appended claims. These and other features and advantages of the invention may be described below in connection with various illustrative embodiments of the invention. The above summary of the present invention is not intended to describe each disclosed embodiment or every implementation of the present invention. The Figures and the detailed description which follow more particularly exemplify illustrative embodiments.

### Brief Description of the Drawings

Preferred embodiments of the invention will now be described in greater detail below with reference to the attached drawings, wherein:
FIG. 1 is a schematic cross-sectional view of one embodiment of a mold apparatus in an open position.
FIG. 2 is a schematic cross-sectional view of one embodiment of a mold apparatus in a closed position.
FIG. 3 is a schematic cross-sectional view of one embodiment of a mold apparatus in a compressed position.
FIG. 4 is a schematic cross-sectional view of one embodiment of a mold apparatus in an open position with an ejected, molded part attached to a polymeric web.
FIG. 5 is a schematic cross-sectional view of one embodiment of a mold apparatus where the polymeric web has been advanced from the position shown in Figure 4.
FIG. 6 is a schematic cross-sectional view of one embodiment of a mold apparatus where a second ejected, molded part is attached to the polymeric web.
FIGS. 7A and 7B are a schematic plan and cross-sectional view, respectively, of one embodiment of a web with integrally affixed microneedle arrays.
FIG. 8 is a schematic plan view of another embodiment of a web with integrally affixed microneedle arrays.
FIG. 9 is a schematic perspective view of a microneedle array.
FIG. 10 is a microphotograph of a microneedle array.

While the above-identified drawing figures set forth several embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of the principles of the invention. The figures may not be drawn to scale. Like reference numbers have been used throughout the figures to denote like parts.

### Detailed Description

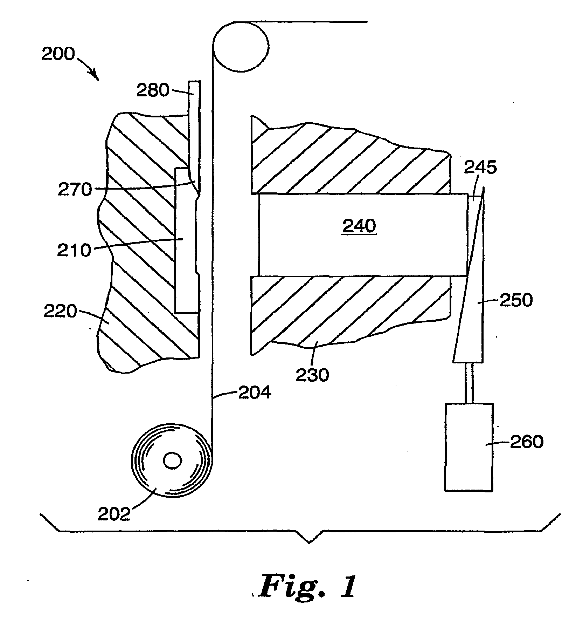

One embodiment of the method of making a molded article is shown in Figures 1 to 4, which illustrate a method for making a molded microneedle array. Figure 1 shows a mold apparatus in the open position. The mold apparatus comprises a mold housing formed from a first mold member 220 and a second mold member 230. The first mold member 220 partially surrounds a mold insert 210 which has the negative image of at least one microneedle. In the illustrated embodiment, the mold insert 210 has the negative image of a microneedle array. The second mold member 230 partially surrounds a compression core 240, which is conventionally in the form of a piston. The mold housing is configured to allow a reciprocal motion between the mold insert 210 and the compression core 240. A wedge 250 that is driven by a hydraulic cylinder 260 transmits force to the compression core 240 via a core-wedge connection 245. The connection 245 is shown as a separate piece, but may be integrally formed as part of the compression core 240 or it may be any conventional linkage that can transmit mechanical force based on the motion of the wedge 250. An input line 280 is used to input molten polymeric material through an injection gate 270 and into the mold cavity that is formed when the mold apparatus is in the closed position. An input film roll 202 is used to provide a web 204, a portion of the web being placed within the opening of the mold apparatus. The web has a first carrier surface that faces the mold insert 210 and a second, opposed, back surface.

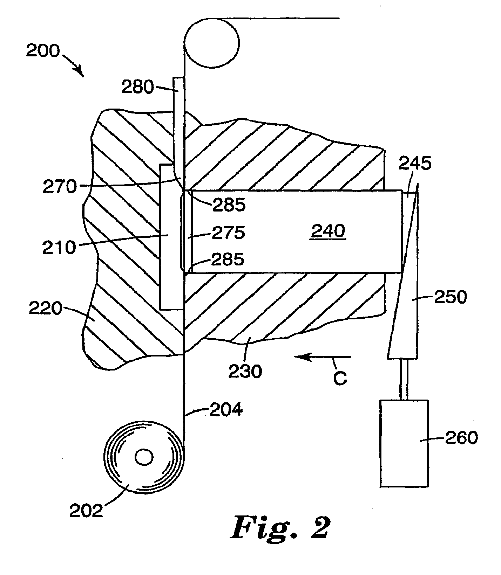

The mold apparatus 200 is closed around the web 204 as shown in Figure 2. The arrow, identified as "C", indicates the direction of motion of the right portion of the apparatus which comprises the second mold member 230, compression core 240, and wedge 250, although it should be noted the orientation of the mold apparatus as shown is arbitrary and may be rotated or inverted as desired. The left portion of the mold apparatus comprises the first mold member 220 and the mold insert 210. In the closed position, the left and right portions of the apparatus are brought together, clamping the web 204 in place and allowing the polymeric material to be injected into the mold apparatus. The left portion and the right portion are spaced apart in the open position, making it possible to remove the molded microneedle from the mold.

The mold apparatus 200 in the closed position defines a mold cavity. The shape of the mold cavity is defined on one major surface by the mold insert 210 and on an opposing major surface by the near end 275 of the compression core 240. The second mold member 230 and the mold insert 210 also define sidewalls 285. The injection gate 270 is an opening on the sidewalls 285. In one embodiment, the sidewalls 285 may be formed entirely by the mold insert 210, that is, the near end 275 of the compression core 240 would be flush with the ends of the second mold member 230. In another embodiment, the sidewalls 285 may be formed entirely by the second mold member 230, that is, the right hand face of the mold insert would be flush with the surface of the first mold member 220. It should be understood that the sidewalls 285 may be formed by any combination of the above descriptions and need not be a separate piece, but are rather intended to define the sides of the mold cavity formed by the interrelation of all of the parts of the mold apparatus. Other designs are equally suitable so long as the mold apparatus defines a mold cavity which may be filled with molten polymeric material under pressure.

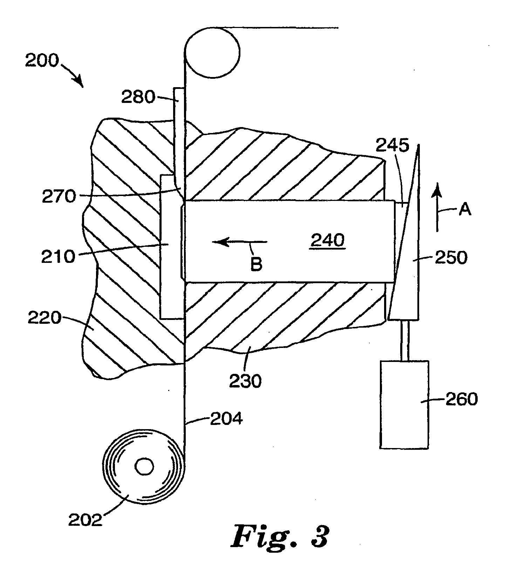

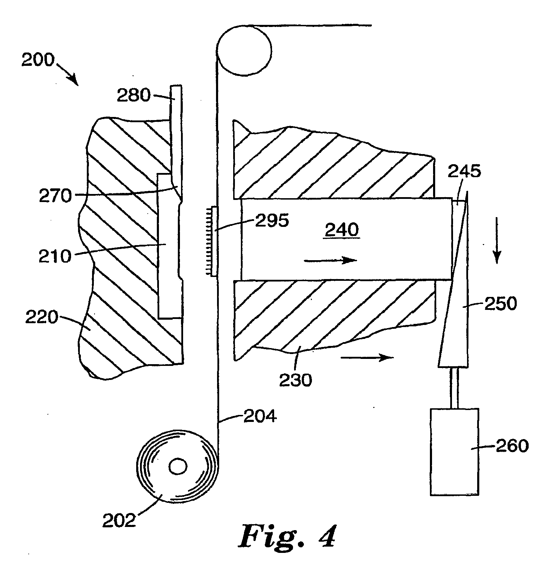

With the mold apparatus in the closed position, molten polymeric material is injected through the input line 280 and injection gate 270 to partially fill the mold cavity. Once the mold cavity is partially filled to a desired amount, then the hydraulic cylinder 260 moves the wedge 250 in the direction of the arrow identified as 'A' in Figure 3 to place the mold apparatus 200 in a compressed position. Movement of the wedge 250 causes corresponding movement of the compression core 240 in the direction of the arrow identified as 'B', that is, there is a reciprocal motion between the compression core 240 and the mold insert 210. The molten polymeric material is thus compressed within the mold cavity thereby aiding in filling the negative image of the microneedle array in the mold insert 210. The web 204 is also pressed by the compression core 240 against the molten polymeric material in the mold cavity, thereby forming a molded part affixed to the first, carrier surface of the web 204. The mold apparatus is subsequently opened, as shown in Figure 4, and the arrows show the direction of motion of the various parts (compression core 240, wedge 250, second mold member 230) returning to their open positions. A molded microneedle array 295 that is integrally attached to the web 204 is then ejected from the open mold apparatus by any conventional method, such as with the use of ejector pins, vacuum assist, undercuts, air pressure assist (all not shown).

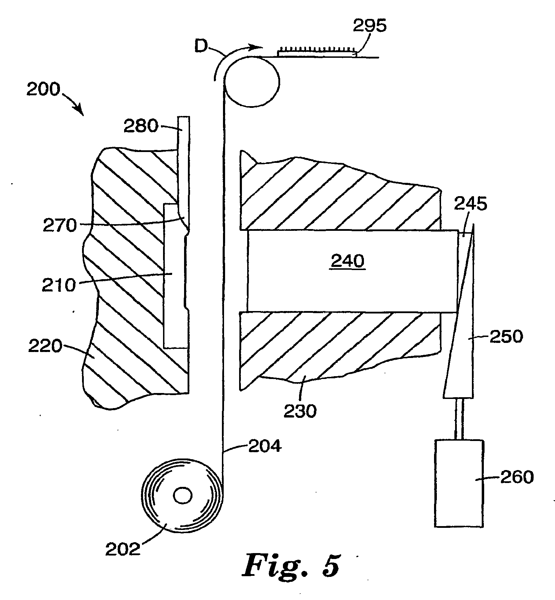

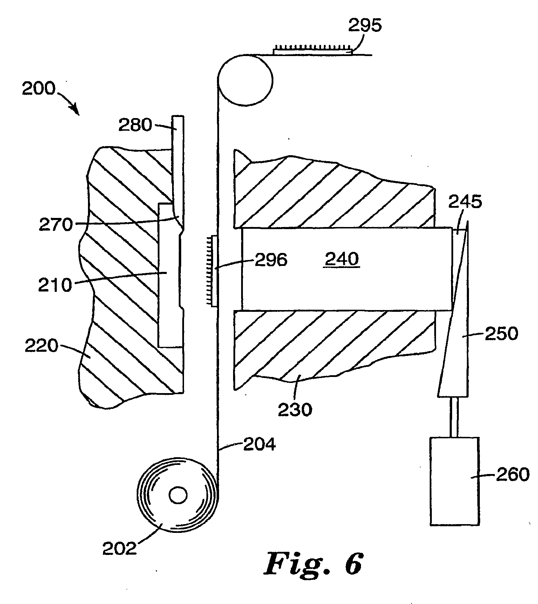

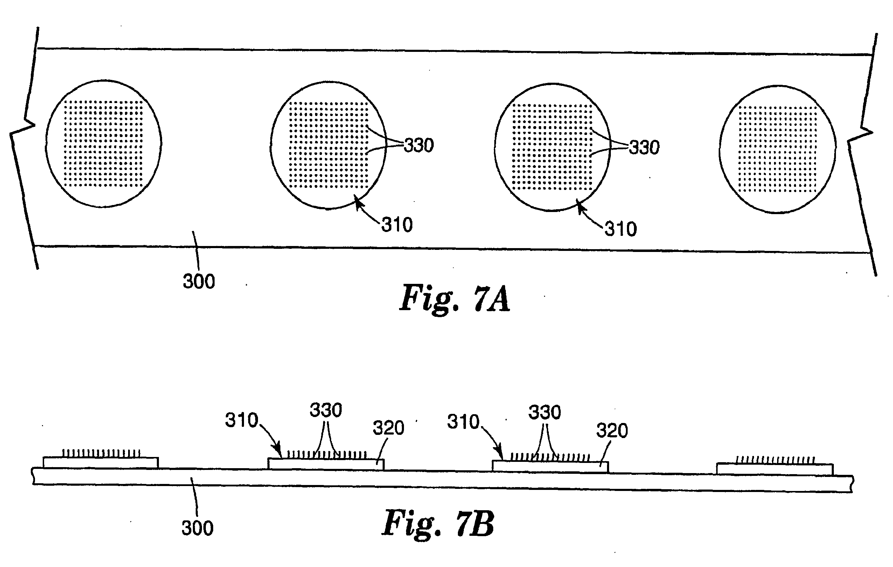

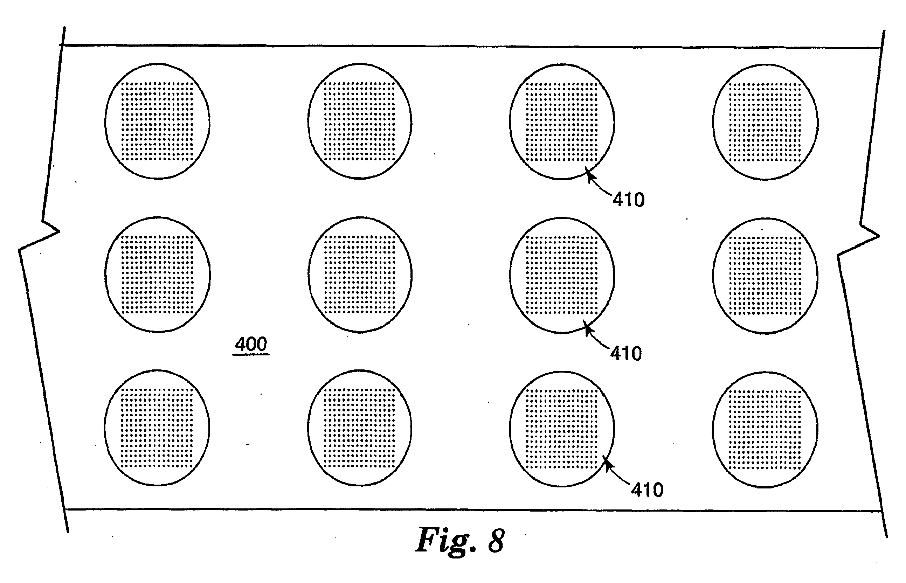

The web 204 may then be advanced in the direction of the arrow identified as 'D' in Figure 5 so that the array 295 that is integrally attached to the web 204 is removed from the mold apparatus 200. Any suitable conventional web-handling equipment, such as unwind and wind-up rolls, idlers, tension bars or rolls, film guides, dancers, laminators, pull rolls, etc., may be used to control the motion of the web. The steps shown in Figures 2 to 4 may then be repeated to provide another array 296 integrally attached to the web 204 as shown in Figure 6. The spacing between arrays may vary and will depend on a number of factors, such as the size of the array, the size of the mold apparatus, the type of polymeric web, the intended use of the arrays, etc. For example, the arrays may be attached to the web in a single row in the longitudinal (or machine) direction of the web or there may be more than one array spaced across the transverse (or cross) direction of the web. The arrays are preferably evenly spaced in one or more directions along the web. Figure 7A shows a plan view and Figure 7B shows a cross-sectional view of a web 300 with arrays 310 evenly spaced in a single row. The arrays 310 are partially patterned with microneedles 330 protruding from the array substrate 320. The array substrate 320 is affixed to the web 300. Figure 8 shows a web 400 with 3 rows of arrays 410 spaced across the transverse direction of the web. The length of the web 400 may generally be indefinite and in practice is only limited by a length that can be suitably handled by conventional web handling methods. For example, a web with arrays (such as that shown in Figure 8) may be wound into a roll form if provided with a protective covering web or liner. Alternatively, it may be desirable to cut the web into discrete sheets for further handling. Such sheets may optionally be protected with a protective covering web or liner. Each sheet may have a convenient number of arrays affixed to the web, preferably in a regular pattern. The portion of the web shown in Figure 8 illustrates a 3 x 4 pattern of arrays attached to the web, but a sheet with any other suitable number of arrays (e.g., 5 x 5, 5 x 10, 10 x 10, etc.) may be prepared.

The mold insert is characterized as having microstructured features, such that the molded article removed from the mold apparatus is characterized as a microstructured molded article. Microstructured features typically have a major dimension (e.g., length, width, height) that is about 500 microns or less in size and sometimes about 250 microns or less in size. Microstructured features typically have a major dimension (e.g., length, width, height) that is about 1 micron or greater in size and sometimes about 25 microns or greater in size. The microstructured features extend away from the carrier surface, that is, the features are generally on an exposed portion of the molded article. The microstructured features may be depressions or cavities in the exposed surface of the molded article, protrusions, such as microneedles, in the exposed surface of the molded article, or some combination thereof. The microstructured features of the parts made with such a mold insert are often quite delicate and easy to damage by inadvertent contact. In one embodiment, the microstructured features are present on a major surface of the molded article that is generally parallel in alignment with the carrier web surface. In one embodiment, the molded article is characterized by a plurality of microstructured features present in a regular pattern.

The web with integrally attached arrays may then be further handled or processed in many different ways. For example, the web may be used as a means to transport arrays to a separate coating station where a pharmaceutical preparation is applied to the surface of the needles. If such a preparation is applied with use of a carrier fluid that is subsequently allowed to evaporate, then the web may further serve to transport the array from the coating station to a drying station, such as an oven. The web with attached arrays may also be used to transport arrays to a converting station where additional components, such as a skin facing adhesive may be added to the array or to the web surrounding the array. The web with attached arrays may be stored for later use or processing (e.g., with the aid of a covering surface or liner to protect the integrity of the microneedles).

In one embodiment, the web with attached arrays may be used directly in an end use-product, for example, a strip of web with a plurality of arrays arranged in a single row may be loaded into a suitable application device having an advancement feature to allow each array to be applied to a skin surface in succession. In one embodiment, each array may simply be applied and directly removed (e.g., in order to pierce the skin in preparation for a separately applied pharmaceutical substance). In another embodiment, the web may be configured (e.g., with perforations surrounding the array) so as to allow the applicator to punch the array free from the web while applying the microneedles to a surface.

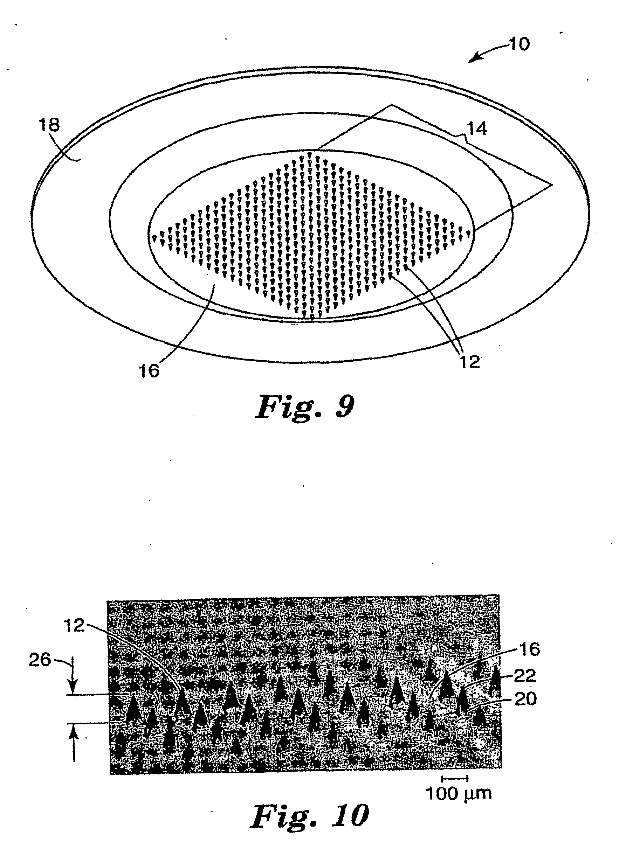

In another embodiment, the web with attached arrays may be used for handling the arrays prior to removing the arrays from the web to provide a plurality of individual arrays. The arrays may be removed from the web by any suitable means, such as by die cutting, punching, or slitting. The arrays may be die-cut from the web, such that the edge of the web is flush with the edge of the array or an additional area of web may be left surrounding the outer edge of the array, as shown and described below in Figure 9. Each individual array may be further combined with other components, such as an adhesive overlay, a retaining collar or other suitable protective packaging, and/or directly mounted into an applicator device suitable for applying the array to a target surface.

Any of a number of conventional types of webs may be used. Webs suitable for use include any substantially continuous material with sufficient integrity to allow for web handling of a finished article having a plurality of molded parts affixed thereto. Substantially continuous web materials should be understood to include metal foils, nonwovens, porous films, paper, woven or knitted cloth, and perforated films, as long as such materials have sufficient continuity to allow them to be handled as a web. In one embodiment, the web is a continuous polymeric film. Examples of suitable continuous polymeric films include polypropylene, polycarbonate, polyethylene, polyimide, and polyester. In one embodiment, the web is selected so as to be able to withstand the heat present in the molding apparatus without losing its integrity. The films will be of a thickness to allow for convenient web handling and will typically be between about (0.2 mil) 5 µm and (50 mil) 1270 µm in thickness, often between about (1 mil) 25 µm and (20 mil) 508 µm in thickness.

In one embodiment, it may be desirable to use the same or a similar material for both the carrier web and the molded article so as to enhance bonding between the molded article and the carrier web. Slides, lifters, or other actions can be used to form undercuts in the molded article and/or assist in pulling the molded article from a mold surface such as a mold insert having many high aspect ratio micro-cavities. Alternatively, tensile forces may be applied directly to the carrier web to assist in pulling the molded article from a mold surface.

In one embodiment the mold apparatus may be configured so as to have multiple, individual mold cavities. Each mold cavity has a negative image of a microneedle array, such that the result of a single cycle of injection and compression produces multiple microneedle arrays. Because the molded articles formed by the individual mold cavities are attached to the carrier web, the use of runners or sprues used in conventional molding processes may be eliminated. The elimination of runners or sprues reduces the amount of material used in forming the molded article and the time required to complete a molding cycle. The number of individual mold cavities may be, for example, 4 or more, often 8 or more, and in some instances 32 or more. The injection pressure with which the molten polymeric material is injected into the mold cavities may be adjusted accordingly depending on the shape, size, and number of cavities being filled. Venting can be accomplished for each individual cavity with grooves in the mold surface on the needle cavity side of the mold to allow air to escape between the web and the mold surface, thus reducing the force needed to fill the mold. The compressive force to the individual mold cavities may be provided by a single device, such as a hydraulic cylinder, which is configured so as to distribute the compressive force evenly across the different cavities. Alternatively, more than one device may be used to supply compressive force. For example, a hydraulic cylinder may be provided to supply compressive force to each mold cavity, to every two mold cavities, or to every 4 mold cavities.

The initial position and motion of the compression core 240 in Figures 2 and 3 is shown in an exaggerated fashion for purposes of illustration. In one embodiment, the bulk of the mold cavity is substantially filled prior to compression and the compression step is performed largely to fill the negative images of microneedles 12 in the mold insert 210. The motion of the compression core is generally selected so as to displace a volume similar in size or larger than the volume of the mold cavity that remains unfilled by the initial injection step. In particular, it may be desirable to displace a larger volume in order to compensate for shrinkage of polymeric material in the mold cavity. Displacement of a larger volume may also be desirable in order to account for material that escapes the mold cavity as parting line flash or to account for mold plate deflections. The motion of the compression core and the resulting volume displaced may be adjusted depending on a number of parameters, including the size of the mold cavity, the shape and number of features in the mold cavity, the amount of the mold cavity filled by the initial injection step, and the type of material molded. Since the microneedle image(s) in the mold insert is relatively small both in height and volume, the motion of the compression core, that is the compression stroke, is typically between about (0.001 to 0.010 inches) 25 µm to 250 µm, often between (0.002 to 0.008 inches) 50 µm to 200 µm, and sometimes between (0.003 to 0.006 inches) 75 µm to 150 µm. The web 204 is shown in Figure 2 prior to injection of the molten polymeric material. After injection of the molten polymeric material, but prior to compression, the web will be pressed against the near end 275 of the compression core 240 and thus deformed slightly. Movement of the compression core 240 as shown in Figure 3 will then return the web to its original configuration.

The applied compressive force is typically greater than (5,000 psi) 345,00 kPa, sometimes greater than (30,000 psi) 207,000 kPa and often greater than (60,000 psi) 414,000 kPa. Additional details regarding injection-compression molding may be found in U. S. Patent Nos. 4,489,033 (Uda et al.), 4,515,543 (Hamner), and 6,248,281 (Abe et al.).

Although the compressive force is supplied by a wedge in the illustrated embodiment, any known conventional method of applying force may be used to provide compressive force to the mold cavity. The compression core may have any suitable shape that forms a major surface of the mold cavity and allows for application of compressive force to the material in the mold cavity. The compression core may be in the form of a piston or pin, and desirably the face of the piston or pin is the same diameter as the part to be formed. One skilled in the art would appreciate that many conventional methods for applying force may be utilized, such as, for example, using a hydraulic pancake cylinder.

The use of compression as an aid for the injection molding process described with regards to Figures 1 to 6 is optional and any other suitable injection molding process may also be used in the present invention, such as those disclosed in U. S. Patent Application Serial No. 60/546,780 and U. S. Patent No. 5,376,317 (Maus et al.).

In another embodiment, the first mold member 220 and mold insert 210 may be heated and cooled using a mold temperature control system. Mold temperature thermal cycling allows for precise control of the internal mold cavity temperature during filling and packing of the soft polymeric material and during ejection of the solid array 295. The media used to either heat or cool the mold may be in the form of oil, water or high pressure steam. In one embodiment, the mold temperature with which the molten material is injected into the mold cavity may be adjusted before or during the mold filling, packing and part ejection stages. The mold temperature during filling and packing of the soft polymeric material is typically greater than (250 °F) 120 °C, sometimes greater than (300 °F) 150 °C, and often greater than (350 °F) 175 °C. The mold temperature during ejection of the array 295 is typically greater than (150 °F) 65 °C, sometimes greater than (200 °F) 95 °C, and often greater than (250 °F) 120 °C.

A wide variety of polymeric materials may be suitable for use as the injected polymeric material. In one embodiment, the material is selected so that it is capable of forming relatively rigid and tough microneedles that resist bending or breaking when applied to a skin surface. In one aspect, the polymeric material has a melt-flow index greater than about 5 g/10 minutes when measured by ASTM D1238 at conditions of 300 °C and 1.2 kg weight. The melt-flow index is often greater than or equal to about 10 g/10 minutes and sometimes greater than or equal to about 20 g/10 minutes. In another embodiment, the tensile elongation at break as measured by ASTM D638 (2.0 in/minute) (50,8 mm/min) is greater than about 100 percent. In still another embodiment, the impact strength as measured by ASTM D256, "Notched Izod", (73°F) (22,77°C) is greater than about 2.67 J/cm (5 ft-lb/inches). Examples of suitable materials include polycarbonate, polyetherimide, polyethylene terephthalate, and mixtures thereof. In one embodiment the material is polycarbonate.

In one embodiment, microneedle devices with molded microneedles integrally formed with a substrate that is affixed to a backing web may be prepared. Figure 9 shows such a microneedle device 10. A portion of the device 10 is illustrated with microneedles 12 protruding from a microneedle substrate surface 16 on the patient-facing portion of the device. The substrate surface 16 is a raised central portion of the device 10 that is integrally affixed to a backing film 18. The exposed outer portion of the backing web 18 on the patient-facing portion of the device may be partially or fully coated with an adhesive to facilitate adherence of the device to a skin surface. The microneedles 12 may be arranged in any desired pattern 14 or distributed over the substrate surface 16 randomly. As shown, the microneedles 12 are arranged in uniformly spaced rows placed in a rectangular arrangement. In one embodiment, the area having microneedles 12 on the patient-facing surface of the device 10 is more than about 0.1 cm² and less than about 20 cm², and in some instances more than about 0.5 cm² and less than about 5 cm². In the embodiment shown in Figure 9, a portion of the substrate surface 16 is non-patterned. In one embodiment, the non-patterned surface has an area of more than about 1 percent and less than about 75 percent of the total area of the device surface that faces a skin surface of a patient. In one embodiment, the non-patterned surface has an area of more than about (0.10 square inch) 0.65 cm² to less than about (1 square inch) 6.5 cm². In another embodiment (not shown), the microneedles are disposed over substantially the entire surface area of the substrate 16. The thickness of the substrate surface may vary depending on the desired end use of the microneedle array. In one embodiment, the substrate surface may be less than (200 mil) 0.51 cmin thickness, often less than (100 mil) 0.25 cm in thickness, and sometimes less than (50 mil) 0.13 cm in thickness. The substrate surface is typically more than (1 mil) 25.4 µm in thickness, often more than (5 mil) 127 µm in thickness, and sometimes more than (10 mil) 203 µm in thickness.

The microneedles are typically less than 1000 microns in height, often less than 500 microns in height, and sometimes less than 250 microns in height. The microneedles are typically more than 5 microns in height, often more than 25 microns in height, and sometimes more than 100 microns in height.

The microneedles may be characterized by an aspect ratio. As used herein, the term "aspect ratio" is the ratio of the height of the microneedle (above the surface surrounding the base of the microneedle) to the maximum base dimension, that is, the longest straight-line dimension that the base occupies (on the surface occupied by the base of the microneedle). In the case of a pyramidal microneedle with a rectangular base, the maximum base dimension would be the diagonal line connecting opposed corners across the base. Microneedles typically have an aspect ratio of between about 2:1 to about 6:1 and sometimes between about 2.5:1 to about 4:1.

The microneedle arrays prepared according to any of the foregoing embodiments may comprise any of a variety of configurations, such as those described in the following patents and patent applications. One embodiment for the microneedle devices comprises the structures disclosed in U. S. Patent Application Publication No. 2003/0045837. The disclosed microstructures in the aforementioned patent application are in the form of microneedles having tapered structures that include at least one channel formed in the outside surface of each microneedle. The microneedles may have bases that are elongated in one direction. The channels in microneedles with elongated bases may extend from one of the ends of the elongated bases towards the tips of the microneedles. The channels formed along the sides of the microneedles may optionally be terminated short of the tips of the microneedles. The microneedle arrays may also include conduit structures formed on the surface of the substrate on which the microneedle array is located. The channels in the microneedles may be in fluid communication with the conduit structures. Another embodiment for the microneedle devices comprises the structures disclosed in co-pending U. S. Patent Application Publication No. 2005/0261631 which describes microneedles having a truncated tapered shape and a controlled aspect ratio. Still another embodiment for the microneedle arrays comprises the structures disclosed in U. S. Patent No. 6,313,612 (Sherman, et al.) which describes tapered structures having a hollow central channel. Still another embodiment for the microneedle arrays comprises the structures disclosed in International Publication No. WO 00/74766 (Gartstein, et al.) which describes hollow microneedles having at least one longitudinal blade at the top surface of tip of the microneedle.

Referring to Figure 10, each of the microneedles 12 includes a base 20 on the substrate surface 16, with the microneedle terminating above the substrate surface in a tip 22. Although the microneedle base 20 shown in Figure 10 is rectangular in shape, it will be understood that the shape of the microneedles 12 and their associated bases 20 may vary with some bases, e.g., being elongated along one or more directions and others being symmetrical in all directions. The base 20 may be formed in any suitable shape, such as a square, rectangle, or oval. In one embodiment the base 20 may have an oval shape (i.e., that is elongated along an elongation axis on the substrate surface 16).

One manner in which the microneedles of the present invention may be characterized is by height 26. The height 26 of the microneedles 12 may be measured from the substrate surface 16. It may be preferred, for example, that the base-to-tip height of the microneedles 12 be about 500 micrometers or less as measured from the substrate surface 16. Alternatively, it may be preferred that the height 26 of the microneedles 12 is about 250 micrometers or less as measured from the base 20 to the tip 22. It may also be preferred that the height of molded microneedles is greater than about 90%, and more preferably greater than about 95%, of the height of the microneedle topography in the mold insert. The microneedles may deform slightly or elongate upon ejection from the mold insert. This condition is most pronounced if the molded material has not cooled below its softening temperature, but may still occur even after the material is cooled below its softening temperature. It is preferred that the height of the molded microneedles is less than about 115%, and more preferably less than about 105%, of the height of the microneedle topography in the mold.

The general shape of the microneedles of the present invention may be tapered. For example, the microneedles 12 may have a larger base 20 at the substrate surface 16 and extend away from the substrate surface 16, tapering to a tip 22. In one embodiment the shape of the microneedles is pyramidal. In another embodiment, the shape of the microneedles is generally conical. In one embodiment the microneedles have a defined tip bluntness, such as that described in co-pending and commonly owned U.S. Patent Application Publication No. 2005/0261631, wherein the microneedles have a flat tip comprising a surface area measured in a plane aligned with the base of about 20 square micrometers or more and 100 square micrometers or less. In one embodiment, the surface area of the flat tip will be measured as the cross-sectional area measured in a plane aligned with the base, the plane being located at a distance of 0.98h from the base, where h is the height of the microneedle above the substrate surface measured from base to tip.

In one embodiment, the negative image(s) of the at least one microneedle is substantially completely filled with injected polymeric material prior to opening the mold and ejecting the part. By substantially completely filled, it should be understood that the molded microneedle should have a height greater than about 90 percent of the corresponding height of the microneedle topography in the mold insert. In one embodiment, the molded microneedle has a height greater than about 95 percent of the corresponding height of the microneedle topography in the mold insert. It is preferable that the molded microneedle has a height substantially the same (e.g., 95 percent to 105 percent) as the corresponding height of the microneedle topography in the mold insert.

Mold inserts suitable for use in the present invention may be made by any known conventional method. In one method, a positive 'master' is used to form the mold insert. The positive master is made by forming a material into a shape in which the microneedle array will be molded. This master can be machined from materials that include, but are not limited to, copper, steel, aluminum, brass, and other heavy metals. The master can also be made from thermoplastic or thermoset polymers that are compression formed using silicone molds. The master is fabricated to directly replicate the microneedle array that is desired. The positive master may be prepared by a number of methods and may have microneedles of any of a variety of shapes, for example, pyramids, cones, or pins. The protrusions of the positive master are sized and spaced appropriately, such that the microneedle arrays formed during molding using the subsequently formed mold insert have substantially the same topography as the positive master.

A positive master may be prepared by direct machining techniques such as diamond turning, disclosed in U. S. Patent No. 5,152,917 (Pieper, et al.) and U. S. Patent No. 6,076,248 (Hoopman, et al.). A microneedle array can be formed in a metal surface, for example, by use of a diamond turning machine, from which is produced a mold insert having an array of cavity shapes. The metal positive master can be manufactured by diamond turning to leave the desired shapes in a metal surface which is amenable to diamond turning, such as aluminum, copper or bronze, and then nickel plating the grooved surface to provide the metal master. A mold insert made of metal can be fabricated from the positive master by electroforming. These techniques are further described in U.S. Patent No. 6,021,559 (Smith).

Microneedle arrays prepared by methods of the present invention may be suitable for delivering drugs (including any pharmacological agent or agents) through the skin in a variation on transdermal delivery, or to the skin for intradermal or topical treatment, such as vaccination.

In one aspect, drugs that are of a large molecular weight may be delivered transdermally. Increasing molecular weight of a drug typically causes a decrease in unassisted transdermal delivery. Microneedle devices suitable for use in the present invention have utility for the delivery of large molecules that are ordinarily difficult to deliver by passive transdermal delivery. Examples of such large molecules include proteins, peptides, nucleotide sequences, monoclonal antibodies, DNA, polysaccharides, such as heparin, and antibiotics, such as ceftriaxone.

In another aspect, microneedle arrays prepared by methods of the present invention may have utility for enhancing or allowing transdermal delivery of small molecules that are otherwise difficult or impossible to deliver by passive transdermal delivery. Examples of such molecules include salt forms; ionic molecules, such as bisphosphonates, preferably sodium alendronate or pamedronate; and molecules with physicochemical properties that are not conducive to passive transdermal delivery.

In another aspect, microneedle arrays prepared by methods of the present invention may have utility for enhancing delivery of molecules to the skin, such as in dermatological treatments, vaccine delivery, or in enhancing immune response of vaccine adjuvants. Examples of suitable vaccines include flu vaccine, Lyme disease vaccine, rabies vaccine, measles vaccine, mumps vaccine, chicken pox vaccine, small pox vaccine, hepatitis vaccine, pertussis vaccine, rubella vaccine, diphtheria vaccine, encephalitis vaccine, yellow fever vaccine, recombinant protein vaccine, DNA vaccine, polio vaccine, therapeutic cancer vaccine, herpes vaccine, pneumococcal vaccine, meningitis vaccine, whooping cough vaccine, tetanus vaccine, typhoid fever vaccine, cholera vaccine, tuberculosis vaccine, and combinations thereof. The term "vaccine" thus includes, without limitation, antigens in the forms of peptides, proteins, polysaccarides, oligosaccarides, DNA, or weakened or killed viruses. Additional examples of suitable vaccines and vaccine adjuvants are described in United States Patent Application Publication No. 2004/0049150.

Microneedle devices may be used for immediate delivery, that is where they are applied and immediately removed from the application site, or they may be left in place for an extended time, which may range from a few minutes to as long as 1 week. In one aspect, an extended time of delivery may be from 1 to 30 minutes to allow for more complete delivery of a drug than can be obtained upon application and immediate removal. In another aspect, an extended time of delivery may be from 4 hours to 1 week to provide for a sustained release of drug. In one aspect, the drug may be applied to the skin (e.g., in the form of a solution that is swabbed on the skin surface or as a cream that is rubbed into the skin surface) prior to applying the microneedle device.

## Claims

1. A method of making injection molded microstructured drug delivery or tissue fluid sampling articles on a carrier web (204) comprising the steps of:
(a) providing a web having a first, carrier surface and a second, back surface;
(b) providing a mold apparatus (200) comprising an injection gate (270) and a mold insert (210) having microstructured features, wherein the mold apparatus has an open position and a closed position;
(c) placing the mold apparatus (200) in the open position;
(d) placing a portion of the web (204) within an opening in the mold apparatus (200);
(e) closing the mold apparatus (200) on the web (204);
(f) injecting polymeric material through the injection gate (270) into the closed mold apparatus (200) to form a molded part (295) that is affixed to the first, carrier surface of the web (204), wherein the molded part (295) has microstructured features extending away from the carrier surface;
(g) opening the mold apparatus (200) and removing the molded part (295) from the mold insert (210); and
(h) advancing the web (204) such that the molded part (295) is moved outside of the mold apparatus (200) and another portion of the web (204) is placed within the opening in the mold apparatus (200).

2. A method as claimed in claim 1 wherein the mold insert (210) has the negative image of at least one microneedle (330).

3. A method as claimed in claim 2 wherein the mold insert has the negative image of a plurality of microneedles (330) in the form of an array.

4. A method as claimed in claim 3 wherein the molded part is a microneedle array.

5. A method of making a microneedle device comprising the steps of:
(a) preparing a microneedle array as claimed in claim 4; and
(b) cutting or punching the array from the web (204) to provide a microneedle device attached to a web backing.

6. A method as claimed in claim 1 wherein the web (204) is a substantially continuous film.

7. A method as claimed in claim 1 wherein the web (204) is a polymeric film.

8. A method as claimed in claim 6 wherein, after step (h) is completed, steps (e) to (h) are repeated thereby providing a web (204) with a plurality of molded parts (295) integral with the film.

9. A method as claimed in any preceding claim wherein the mold apparatus comprises a mold chamber having one side defined by an exposed microstructured surface of the mold insert (210).

10. A method as claimed in any preceding claim and further including a step of compressing the injected polymeric material between the mold insert (210) and a compression core (240) by a reciprocal motion between the compression core (240) and the mold insert (210).

11. A method as claimed in any preceding claim wherein the mold apparatus further comprises sidewalls having an injection gate (270) and the polymeric material is injected through the injection gate (270) into the closed mold apparatus (200).

12. A method as claimed in claim 11 wherein the sidewalls further comprise an overflow vent.

## Patentansprüche

1. Verfahren des Herstellens spritzgeformter mikrostrukturierter Wirkstoffabgabe- oder Gewebeflüssigkeitprobennahmegegenstände auf einem Trägernetz (204), welches die folgenden Schritte aufweist:
(a) Bereitstellen eines Netzes, das eine erste Trägerfläche und zweite Rückfläche aufweist;
(b) Bereitstellen eines Formapparates (200), der eine Einspritzöffnung (270) und einen Formeinsatz (210) mit mikrostrukturierten Merkmalen aufweist, wobei der Formapparat eine offene Position und eine geschlossene Position aufweist;
(c) Platzieren des Formapparats (200) in der offenen Position;
(d) Platzieren eines Abschnitts des Netzes (204) in eine Öffnung in dem Formapparat (200);
(e) Schließen des Formapparats (200) auf dem Netz (204);
(f) Einspritzen von polymerem Material durch die Einspritzöffnung (270) in den geschlossenen Formapparat (200), um ein geformtes Teil (295) zu bilden, das an der ersten Trägerfläche des Netzes (204) befestigt ist, wobei das geformte Teil (295) mikrostrukturierte Merkmale aufweist, die von der Trägerfläche weg verlaufen;
(g) Öffnen des Formapparats (200) und Entnehmen des geformten Teils (295) aus dem Formeinsatz (210); und
(h) derartiges Vorschieben des Netzes (204), dass das geformte Teil (295) aus dem Formapparat (200) heraus bewegt wird und ein anderer Abschnitt des Netzes (204) in der Öffnung des Formapparats (200) platziert wird.

2. Verfahren nach Anspruch 1, wobei der Formeinsatz (210) das Negativbild mindestens einer Mikronadel (330) aufweist.

3. Verfahren nach Anspruch 2, wobei der Formeinsatz das Negativbild mehrerer Mikronadeln (330) in der Form einer Anordnung aufweist.

4. Verfahren nach Anspruch 3, wobei es sich bei dem geformten Teil um eine Mikronadel-Anordnung handelt.

5. Verfahren des Herstellens einer Mikronadel-Vorrichtung, das die folgenden Schritte aufweist:
(a) Herstellen einer Mikronadel-Anordnung nach Anspruch 4; und
(b) Schneiden oder Stanzen der Anordnung aus dem Netz (204), um eine an einer Netzverstärkung angebrachte Mikronadel-Vorrichtung bereitzustellen.

6. Verfahren nach Anspruch 1, wobei es sich bei dem Netz (204) um einen im Wesentlichen durchgängigen Film handelt.

7. Verfahren nach Anspruch 1, wobei es sich bei dem Netz (204) um einen polymeren Film handelt.

8. Verfahren nach Anspruch 6, wobei nach Beendigung von Schritt (h) Schritte (e) bis (h) wiederholt werden, um dadurch ein Netz (204) mit mehreren geformten Teilen (295) integral mit dem Film bereitzustellen.

9. Verfahren nach einem der vorhergehenden Ansprüche, wobei der Formapparat eine Formkammer aufweist, die eine Seite aufweist, welche von einer freiliegenden mikrostrukturierten Fläche des Formeinsatzes (210) definiert ist.

10. Verfahren nach einem der vorhergehenden Ansprüche und ferner aufweisend einen Schritt des Komprimierens des eingespritzten polymeren Materials zwischen dem Formeinsatz (210) und einem Kompressionskern (240) durch eine wechselseitige Bewegung zwischen dem Kompressionskern (240) und dem Formeinsatz (210).

11. Verfahren nach einem der vorhergehenden Ansprüche, wobei der Formapparat ferner Seitenwände aufweist, welche eine Einspritzöffnung (270) aufweisen, und das polymere Material durch die Einspritzöffnung (270) in den geschlossenen Formapparat (200) gespritzt wird.

12. Verfahren nach Anspruch 11, wobei die Seitenwände ferner einen Überlaufschlitz aufweisen.

## Revendications

1. Procédé de fabrication d'articles microstructurés moulés par injection destinés à l'administration de médicaments ou à l'échantillonnage de liquides tissulaires sur une bande de support (204), consistant à :
(a) fournir une bande comportant une première surface qui est une surface de support et une deuxième surface qui est une surface postérieure ;
(b) fournir un appareil sous forme de moule (200) comprenant une entrée d'injection (270) et un insert de moule (210) comportant des caractéristiques microstructurées, l'appareil sous forme de moule ayant une position ouverte et une position fermée ;
(c) placer l'appareil sous forme de moule (200) en position ouverte ;
(d) placer une partie de la bande (204) à l'intérieur d'une ouverture dans l'appareil sous forme de moule (200) ;
(e) fermer l'appareil sous forme de moule (200) sur la bande (204) ;
(f) injecter un matériau polymère dans l'appareil sous forme de moule (200) fermé, par l'intermédiaire de l'entrée d'injection (270), pour former une pièce moulée (295) qui est fixée à la première surface qui est une surface de support de la bande (204), la pièce moulée (295) comportant des caractéristiques microstructurées qui s'étendent dans une direction opposée à la surface de support ;
(g) ouvrir l'appareil sous forme de moule (200) et retirer la pièce moulée (295) de l'insert de moule (210) ; et
(h) faire avancer la bande (204) de façon à déplacer la pièce moulée (295) en la faisant sortir de l'appareil sous forme de moule (200) et à positionner une autre partie de la bande (204) à l'intérieur de l'ouverture de l'appareil sous forme de moule (200).

2. Procédé selon la revendication 1, dans lequel l'insert de moule (210) porte l'empreinte négative d'au moins une microaiguille (330).

3. Procédé selon la revendication 2, dans lequel l'insert de moule porte l'empreinte négative d'une pluralité de microaiguilles (330) sous la forme d'une matrice.

4. Procédé selon la revendication 3, dans lequel la pièce moulée est une matrice de microaiguilles.

5. Procédé de fabrication d'un dispositif à microaiguilles, comprenant les étapes qui consistent à :
(a) préparer une matrice de microaiguilles selon la revendication 4 ; et
(b) couper ou découper à l'emporte-pièce la matrice pour la retirer de la bande (204) afin de produire un dispositif à microaiguilles fixé à un support sous forme de bande.

6. Procédé selon la revendication 1, dans lequel la bande (204) est un film sensiblement continu.

7. Procédé selon la revendication 1, dans lequel la bande (204) est un film polymère.

8. Procédé selon la revendication 6, dans lequel, après l'exécution de l'étape (h), les étapes (e) à (h) sont répétées, en produisant ainsi une bande (204) pourvue d'une pluralité de pièces moulées (295) faisant corps avec le film.

9. Procédé selon l'une quelconque des revendications précédentes, dans lequel l'appareil sous forme de moule comprend une chambre de moulage comportant un côté délimité par une surface microstructurée exposée de l'insert de moule (210).

10. Procédé selon l'une quelconque des revendications précédentes et comprenant en outre une étape qui consiste à comprimer le matériau polymère injecté entre l'insert de moule (210) et un noyau de compression (240) par un mouvement de va-et-vient entre le noyau de compression (240) et l'insert de moule (210).

11. Procédé selon l'une quelconque des revendications précédentes, dans lequel l'appareil sous forme de moule comprend en outre des parois latérales comportant une entrée d'injection (270) et le matériau polymère est injecté dans l'appareil sous forme de moule (200) fermé par l'intermédiaire de l'entrée d'injection (270).

12. Procédé selon la revendication 11, dans lequel les parois latérales comprennent en outre un trop-plein.
